# EUROPÄISCHE PATENTANMELDUNG

(11) **EP 3 323 797 A1**
(43) Veröffentlichungstag der Anmeldung: **23.05.2018**
(21) Anmeldenummer: 16199921.4
(22) Anmeldetag: 22.11.2016
(51) Int. Cl.: C07C 5/48, C07C 7/10, C07C 7/11, C07C 11/167

(54) **VERFAHREN ZUR HERSTELLUNG VON 1,3-BUTADIEN AUS N-BUTENEN DURCH OXIDATIVE DEHYDRIERUNG UMFASSEND EINE SAURE WÄSCHE DES C4-PRODUKTGASSTROMS**

(71) Anmelder: BASF SE, 67056 Ludwigshafen am Rhein (DE); Linde AG, 80331 München (DE)
(72) Erfinder: BALEGEDDE RAMACHANDRAN, Ragavendra Prasad, 67056 Ludwigshafen (DE); JOSCH, Jan Pablo, 67056 Ludwigshafen (DE); HAMMEN, Oliver, 67056 Ludwigshafen (DE); UNGELENK, Jan, 67056 Ludwigshafen (DE)
(74) Vertreter: Schuck, Alexander

(57) **Zusammenfassung**

1. Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b in einer oder mehreren Abkühlstufen durch Inkontaktbringen mit einem im Kreis geführten Kühlmittel, wobei Wasserdampf und zumindest ein Teil der hochsiedenden Nebenkomponenten kondensieren;
Cb) Kompression des verbleibenden Produktgasstroms b in mindestens einer Kompressionsstufe Cba) und Abkühlung des komprimierten Gasstroms b in mindestens einer Abkühlstufe Cbb) durch Inkontaktbringen mit einem Abkühlmittel, wobei ein wässriges Kondensat c1 b und ein Gasstrom c2 enthaltend Butadien, n-Butene, Wasserdampf, gegebenenfalls Sauerstoff, leicht siedende Kohlenwasserstoffe, gegebenenfalls Kohlenstofffoxide und gegebenenfalls Inertgase erhalten werden;
Da) Abtrennung von nicht kondensierbaren und leicht siedenden Gasbestandteilen umfassend gegebenenfalls Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein Nebenkomponenten enthaltender C₄-Produktgasstrom d1 erhalten wird;
dadurch gekennzeichnet, dass Schritt Cb) eine Waschstufe umfasst, wobei in der Waschstufe aus dem komprimierten Produktgasstrom b durch Waschen mit einer Waschlösung aus Wasser und Essigsäure Aldehyde abgetrennt werden.

## Beschreibung

Die Erfindung betrifft ein Verfahren zur Herstellung von 1,3-Butadien aus n-Butenen durch oxidative Dehydrierung (ODH), bei dem zur Aldehyd-Abtrennung eine saure Wäsche des C₄-Pro-duktgasstroms durchgeführt wird.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (Acrylnitril-Butadien-StyrolCopolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, C₅-und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung (Oxidehydrierung, ODH) von n-Butenen zu Butadien kann jedes beliebige n-Butene enthaltende Gemisch eingesetzt werden. Beispielsweise kann eine Fraktion verwendet werden, die als Hauptbestandteil n-Butene (1-Buten und/oder 2-Buten) enthält und aus der C₄-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

Verfahren zur oxidativen Dehydrierung von Butenen zu Butadien sind grundsätzlich bekannt. Derartige Verfahren umfassen häufig die nachstehenden Schritte:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b durch Inkontaktbringen mit einem Kühlmittel und Kondensation zumindest eines Teils der hochsiedenden Nebenkomponenten;
Cb) Kompression des verbleibenden Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
Da) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

US3,884,650 beschreibt ein Verfahren, bei dem das Produktgas der oxidativen Dehydrierung, das Oxygenate und insbesondere Carbonylverbindungen enthält, in einem Quenchapparat durch direktes Inkontaktbringen mit einem wässrigen Kühlmedium gekühlt wird. Das wässrige Kühlmedium weist dabei einen hohen pH-Wert von >10, bevorzugt 11-12 auf. Dabei werden mindestens 90% der Carbonylverbindungen aus dem Produktgas entfernt. Zur Behandlung des die Carbonylverbindungen enthaltenden Abwassers wird vorgeschlagen, die Carbonylverbindungen in einem Stripper zu entfernen. Es wird auch auf das Problem eingegangen, dass Carbonylverbindungen insbesondere bei hohen Temperaturen zur Polymerisierung neigen und zum Fouling im Stripper führen können. Daher werden die Carbonylverbindungen durch Strippen bei vermindertem Druck aus dem Abwasser entfernt.

US 2012/0130137A1 beschreibt ein Verfahren zur oxidativen Dehydrierung von Butenen zu Butadien unter Verwendung von Katalysatoren, die Oxide von Molybdän, Bismut und in der Regel weiteren Metallen enthalten. In Absatz [0122] wird auch auf die Problematik Nebenprodukte hingewiesen. Insbesondere werden Phthalsäureanhydrid, Anthrachinon und Fluorenon genannt, die typischerweise in Konzentrationen von 0,05 bis 0,10 Vol.-% im Produktgas vorlägen. In der US 2012/0130137A1 Absatz [0124] bis [0126] wird empfohlen, die heißen Reaktoraustragsgase direkt durch Kontakt mit einer Kühlflüssigkeit (Quench-Turm) auf zunächst 5 bis 100°C abzukühlen. Als Kühlflüssigkeiten werden Wasser oder wässrige Alkali-Lösungen genannt. Ausdrücklich wird die Problematik von Verstopfungen im Quench durch Hochsieder aus dem Produktgas oder durch Polymerisationsprodukte hochsiedender Nebenprodukte aus dem Produktgas erwähnt, weshalb es vorteilhaft sei, dass hochsiedende Nebenprodukte so wenig wie möglich aus dem Reaktionsteil in den Kühlungsteil (Quench) ausgetragen werden. Eine Abtrennung von iso-Buten oder von dessen Umsetzungsprodukt Methacrolein, von Acetaldehyd oder von Acrolein wird nicht erwähnt.

In JP 2011-001341A wird für ein Verfahren zur oxidativen Dehydrierung von Alkenen zu konjugierten Alkadienen eine zweistufige Kühlung beschrieben. Dabei wird das Produktaustragsgas der oxidativen Dehydrierung zunächst auf eine Temperatur zwischen 300 und 221 °C eingestellt und dann auf eine Temperatur zwischen 99 und 21 °C weiter abgekühlt. In den Absätzen [0066] ff. wird beschrieben, dass zur Einstellung der Temperatur zwischen 300 und 221 °C bevorzugt Wärmetauscher eingesetzt werden, wobei aber auch ein Teil der Hochsieder aus dem Produktgas in diesen Wärmetauschern ausfallen könnten. In der JP2011-001341A wird deshalb ein gelegentliches Auswaschen von Ablagerungen aus den Wärmetauschern mit organischen oder wässrigen Lösungsmitteln beschrieben. Als Lösungsmittel werden beispielsweise aromatische Kohlenwasserstoffe wie Toluol oder Xylol oder ein alkalisches wässriges Lösungsmittel wie beispielsweise die wässrige Lösung von Natriumhydroxid beschrieben. Um ein zu häufiges Abstellen des Verfahrens zur Reinigung des Wärmetauscher zu vermeiden, wird in JP 2011-001341A ein Aufbau mit zwei parallel angeordneten Wärmetauschern beschrieben, die jeweils abwechselnd betrieben oder gespült werden (sogenannte A/B-Fahrweise). Eine Abtrennung von iso-Buten oder von dessen Umsetzungsprodukt Methacrolein, von Acetaldehyd oder von Acrolein wird nicht erwähnt.

Iso-Buten ist in nahezu allen C₄-Kohlenwasserstoffströmen, die für den ODH-Prozess Verwendung finden können, enthalten. Insbesondere C₄-Kohlenwasserstoffströme aus FCC-Crackern enthalten iso-Buten in Mengen von bis zu 15 Vol.-%. Das in den ODH-Reaktor eintretende iso-Buten wird, abhängig vom verwendeten Katalysator und den Reaktionsbedingungen, zu ca.

50% zu Methacrolein umgesetzt. Dieses reichert sich im Kreislaufstrom des Absorptions-/Desorptionsteil der C₄-Kohlenwasserstoff-Abtrennung an und kann Nebenreaktionen wie Oligomerisierungen und Polymerisationen, Ablagerungen auf den Kolonneneinbauten und insbesondere an Verdampfern und Kondensatoren sowie eine Verschlechterung der Trennleistung verursachen.

Bei der Extraktivdestillation in Schritt E) kann es zur Bildung von Polymeren kommen, wenn der C₄-Kohlenwasserstoffstrom d1 bestimmte Nebenkomponenten wie Acrolein und Methacrolein enthält, die mit Butadien Polymere bilden können.

Eine mögliche Lösung für dieses Problem wird in JP2013103896 und JP2014224070 beschrieben. Dort wird eine Gas-Flüssig-Wäsche des gasförmigen C₄-Produktgasstroms mit Wasser beschrieben. Dabei wird aber nur das physikalische Lösungsvermögen von Wasser für bestimmte Nebenkomponenten ausgenutzt, um diese Nebenkomponenten aus dem C₄-Produkt-gasstrom abzutrennen. Dadurch ist die Wascheffizienz nicht sehr hoch und es werden große Wassermengen benötigt, oder der Abreicherungsgrad ist nicht zufriedenstellend. In dem in dem in JP2013-103896 und JP2014224070 beschriebenen Verfahren werden die Aldehyde aus dem C₄-Produktstrom nach der C₄-Absorptions/Desorptionsstufe abtrennt.

CN103086829 beschreibt ein Verfahren, bei dem das Produktgas der oxidativen Dehydrierung, enthaltend Oxygenate und insbesondere Aldehyde, durch direkten Kontakt mit einem wässrigen Kühlmedium gekühlt wird. Dabei werden große Teile organischer Säuren aus dem Produktgas ausgewaschen. Das verbleibende Aldehyd-haltige Produktgas wird auf 5 bis 20 bar verdichtet und in einen Aldehyd-Waschturm eingeführt und die Aldehyde durch direkten Kontakt mit Wasser im Gegenstrom ausgewaschen. Die Temperatur am Kopf des Aldehyd-Waschturms beträgt 5-40°C und die im Turm 5-60°C. Der Betriebsdruck beträgt 5 bis 20 bar. Der Aldehyd-Waschturm kann eine Bodenkolonne, eine Füllkörperkolonne oder eine Siebbodenkolonne sein.

WO 2013/136434 A1 beschreibt ein Verfahren, bei dem Aldehyde und insbesondere Acetaldehyd aus dem flüssigen C₄-Kohlenwasserstoffstrom hinter der C₄-Kohlenwasserstoffabtrennung durch Extraktion abgetrennt werden. Dabei wird der flüssige C₄-Kohlenwasserstoffstrom mit Wasser in Kontakt gebracht, und die Aldehyde werden in einer Extraktionskolonne aus der Flüssigphase extrahiert. Die wasserlöslichen Komponenten werden durch Destillation abgetrennt und das Wasser wird wieder in die Extraktion zurückgeführt.

WO 2013/148913 A1 beschreibt ein Verfahren, bei dem Aldehyde aus dem C₄-Kohlenwasserstoffstrom nach der zweiten Kompressionsstufe abgetrennt werden. Dabei wird der Produktgasstrom mit Wasser in Kontakt gebracht. Das mit Aldehyden beladene Wasser aus der Waschkolonne wird regeneriert und wiederverwendet.

CN103964997 A beschreibt ein Verfahren, bei dem das Produktgas einer oxidativen Dehydrierung von Butenen nach der Entfernung von organischen Säuren auf 0,5 bis 2 MPa komprimiert und in einer Waschkolonne mit Wasser im Gegenstrom gewaschen wird, um Aldehyde aus dem Gas zu entfernen. Das mit Aldehyden beladene Wasser aus der Waschkolonne wird in einer Abwasserhandlungskolonne behandelt.

Aufgabe der Erfindung ist es, ein verbessertes Verfahren zur Herstellung von Butadien durch oxidative Dehydrierung von n-Butenen und anschließender Aufarbeitung des C₄-Kohlenwasserstoffe und Nebenprodukte enthaltenden Produktgasstroms bereitzustellen, das den oben beschriebenen Nachteilen abhilft.

Gelöst wird die Aufgabe durch ein Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, gegebenfalls Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b in einer oder mehreren Abkühlstufen durch Inkontaktbringen mit einem im Kreis geführten Abkühlmittel, wobei Wasserdampf und zumindest ein Teil der hochsiedenden Nebenkomponenten kondensieren;
Cb) Kompression und des verbleibenden Produktgasstroms b in mindestens einer Kompressionsstufe Cba) und Abkühlung des komprimierten Gasstroms b in mindestens einer Abkühlstufe Cbb) durch Inkontaktbringen mit einem Abkühlmittel, wobei ein wässriges Kondensat c1 b und ein Gasstrom c2 enthaltend Butadien, n-Butene, Wasserdampf, leicht siedende Kohlenwasserstoffe, gegebenenfalls Sauerstoff, Kohlenstoffoxide und gegebenenfalls Inertgase erhalten werden;
Da) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein Nebenkomponenten enthaltender C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
dadurch gekennzeichnet, dass Schritt Cb) eine Waschstufe umfasst, wobei in der Waschstufe aus dem komprimierten Produktgasstrom b durch Waschen mit einer Waschlösung aus Wasser und Essigsäure Aldehyde abgetrennt werden.

Überraschend wurde festgestellt, dass die Entfernung von Aldehyden durch Waschen mit einem Wasser/Essigsäure-Gemisch besonders gut gelingt. Die Wäsche erfolgt an dem komprimierten Produktgasstrom b vor Durchführung des Absorptionsschrittes Da).

Im Allgemeinen enthält die in Schritt Cb) eingesetzte Waschlösung 30 bis 95 Gew.-% Wasser und 5 bis 70 Gew.-% Essigsäure, bevorzugt 30 bis 70 Gew.-% Wasser und 30 bis 70 Gew.-% Essigsäure und besonders bevorzugt 40 bis 60 Gew.-% Wasser und 40 bis 60 Gew.-% Essigsäure.

Die Stufe Cb) kann mehrere Kompressionsstufen Cba1) bis Cban) und mehrere Abkühlstufen Cbb1) bis Cbbn) umfassen. Dabei folgt im Allgemeinen auf jede Kompressionsstufe eine Abkühlstufe. In einer bevorzugten Ausführungsform ist eine der Abkühlstufen Cbb1) bis Cbbn) als Waschstufe (Wasch- und Abkühlstufe) ausgestaltet. Die Waschstufe kann aber auch zusätzlich zu den Abkühlstufen Cbb1) bis Cbbn) vorhanden sein. In einer anderen Ausführungsform sind 2 oder mehrere oder alle Abkühlstufen als Waschstufen (Wasch- und Abkühlstufen) ausgestaltet. Die Waschstufen können aber auch zusätzlich zu den jeweiligen Abkühlstufen Cbb1) bis Cbbn) vorhanden sein.

In einer besonders bevorzugten Ausführungsformumfasst die Stufe Cb) drei Kompressionsstufen Cba1) bis Cba3) und drei Abkühlstufen Cbb1) bis Cbb3), wobei die mittlere Abkühlstufe Cbb2) als Waschstufe ausgestaltet ist.

In den Abkühlstufen Cbb), die nicht als Waschstufen ausgebildet sind, wird vorzugsweise ein aromatischer Kohlenwasserstoff als Abkühlmittel eingesetzt. Insbesondere wird hierzu Mesitylen eingesetzt.

Die die Waschstufe verlassende, mit Aldehyden beladene Waschlösung wird im Allgemeinen durch Strippen mit einem Inertgas, vorzugsweise Stickstoff, regeneriert. Auch Strippen mit Wasserdampf ist möglich. Die Waschstufe ist im Allgemeinen als Waschkolonne ausgebildet.

Bei 2 oder mehreren Waschstufen kann die beladene Waschlösung jeweils einzeln regeneriert oder 2 oder mehrere beladene Waschlösungen zuerst vereinigt und dann der vereinigte Strom regeneriert werden.

Bei den Aldehyden handelt es sich im Allgemeinen um eine oder mehrere Komponenten aus der Gruppe bestehend aus Acetaldehyd, Acrolein und Methacrolein.

Der Aldehydgehalt im Produktgasstrom b vor Eintritt in die Waschstufe beträgt im Allgemeinen 1000 bis 10 000 ppm. Beim Austritt des Produktgasstroms b aus der Waschstufe beträgt der Aldheydgehalt im Allgemeinen noch 10 bis 500 ppm, bevorzugt 100 bis 300 ppm. Im Allgemeinen erfolgt eine Abreicherung der Aldehyde in dem Produktgasstrom um mindestens einen Faktor 10.

Die in Schritt Cb) eingesetzte Waschkolonne weist im Allgemeinen 5 bis 10 theoretische Stufen auf. Das Massenverhältnis Waschlösung zu C₄-Produktgasstrom im Zulauf der Waschzone beträgt im Allgemeinen 1 : 1 bis 10 : 1. Die Waschkolonne wird vorzugsweise bei einer Sumpftemperatur im Bereich von 30 bis 100°C, insbesondere bei einer Temperatur im Bereich von 30 bis 60°C, einer Kopftemperatur im Bereich von 10 bis 50 °C, insbesondere im Bereich von 20 bis 30°C und einem Druck im Bereich von 2 bis 10 bar, insbesondere im Bereich von 4 bis 7 bar betrieben. Am Kolonnensumpf wird ein mit den Aldehyden beladener Waschlösungsstrom erhalten. Der an Nebenkomponenten abgereicherte C₄-Produktgasstrom wird am Kopf der Waschkolonne erhalten. Drücke sind, soweit nicht anders angegeben, immer Absolutdrücke.

Vorzugsweise wird von dem mit Nebenkomponenten beladenen Waschlösungsstrom durch Strippen mit einem Strippgas ein die Aldehyde enthaltender Strippgasstrom abgetrennt und ein regenerierter Strom aus Waschlösungsmittel zurückgewonnen. Der regenerierte Waschlösungsstrom wird im Allgemeinen zumindest teilweise in den Waschschritt zurückgeführt.

Die dabei eingesetzte Strippkolonne weist im Allgemeinen 5 bis 15 theoretische Stufen auf. Das Massenverhältnis Strippgas zu Waschlösungsmittel beträgt im Allgemeinen 0,1 : 1 bis 0,03 : 1. Die Strippkolonne wird vorzugsweise bei einer Sumpftemperatur im Bereich von 40 bis 130°C, insbesondere bei einer Temperatur im Bereich von 100 bis 120°C, einer Kopftemperatur im Bereich von 50 bis 100°C, insbesondere im Bereich von 80 bis 95°C und einem Druck im Bereich von 1 bis 3 bar, insbesondere im Bereich von 1,5 bis 3 bar betrieben. Der Strippgasstrom, der die Aldehyde und in geringen Mengen C₄-Kohlenwasserstoffe enthält, wird am Kopf der Strippkolonne erhalten.

In der Abkühlstufe Ca) wird ein organisches Lösungsmittel verwendet. Organische Lösungsmittel weisen im Allgemeinen ein sehr viel höheres Lösungsvermögen für die hochsiedenden Nebenprodukte, die in den dem ODH-Reaktor nachgelagerten Anlageteilen zu Ablagerungen und Verstopfungen führen können, als Wasser oder alkalisch-wässrige Lösungen auf. Bevorzugte als Kühlmittel eingesetzte organische Lösungsmittel sind aromatische Kohlenwasserstoffe, beispielsweise Toluol, o-Xylol, m-Xylol, p-Xylol, Diethylbenzole, Trietylbenzole, Diisopropylbenzole, Triisopropylbenzole und Mesitylen oder Gemische daraus. Besonders bevorzugt ist Mesitylen.

Nachstehende Ausführungsformen sind bevorzugte oder besonders bevorzugte Varianten des erfindungsgemäßen Verfahrens.

Die Stufe Ca) wird mehrstufig in Stufen Ca1) bis Can), bevorzugt zweistufig in zwei Stufen Ca1) und Ca2) durchgeführt. Dabei wird besonders bevorzugt zumindest ein Teil des Lösungsmittels nach Durchlaufen der zweiten Stufe Ca2) als Abkühlmittel der ersten Stufe Ca1) zugeführt.

Die Stufe Cb) umfasst mindestens eine Kompressionsstufe Cba) und mindestens eine Abkühlstufe Cbb). Bevorzugt umfasst die Stufe Cb) mindestens zwei Kompressionsstufen und mindestens eine Abkühlstufe, die nicht als Waschstufe ausgebildet ist. Besonders bevorzugt enthält das Abkühlmittel der Abkühlstufen, die nicht als Waschstufen ausgebildet sind, das gleiche organische Lösungsmittel, das in der Stufe Ca) als Abkühlmittel verwendet wird. In einer Variante wird zumindest ein Teil dieses Abkühlmittels nach Durchlaufen der mindestens einen Abkühlstufe Cbb) als Abkühlmittel der Stufe Ca) zugeführt.

Bevorzugt umfasst die Stufe Cb) mehrere Kompressionsstufen Cba1) bis Cban) und Abkühlstufen Cbb1) bis Cbbn), besonders bevorzugt drei Kompressionsstufen Cba1) bis Cba3) und drei Abkühlstufen Cbb1) bis Cbb3), von denen eine Abkühlstufe, vorzugsweise die mittlere, als Wasch- und Abkühlstufe ausgebildet ist. Dabei folgt im Allgemeinen auf jede Kompressionsstufe eine Abkühlstufe.

In einer weiteren Variante umfasst die Stufe Cb) mindestens eine separate Waschstufe und eine separate Abkühlstufe, wobei die separate Waschstufe direkt vor oder direkt nach der separaten Abkühlstufe durchgeführt wird.

Bevorzugt umfasst Schritt D) die Schritte Da1), Da2) und Db):
Da1) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Da2) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Db) Desorption der C₄-Kohlenwasserstoffe aus dem beladenen Absorptionsmittelstrom, wobei ein C₄-Produktgasstrom d1 erhalten wird, der im Wesentlichen aus C₄-Kohlenwasserstoffen besteht und weniger als 100 ppm Sauerstoff umfasst.

Bevorzugt ist das in Schritt Da) eingesetzte hochsiedende Absorptionsmittel ein aromatisches Kohlenwasserstofflösungsmittel, besonders bevorzugt ist das in Schritt Ca) eingesetzte aromatische Kohlenwasserstofflösungsmittel, insbesondere Mesitylen. Verwendet werden können auch Diethylbenzole, Trietylbenzole, Diisopropylbenzole und Triisopropylbenzole.

In einer Ausführungsform der Erfindung wird der in Schritt Da) enthaltene Gasstrom d2 zu mindestens 30 %, bevorzugt zu mindestens 40 % in den Schritt B) zurückgeführt. Das kann dann sinnvoll sein, wenn nur ein geringer Purgestrom aus dem Gasstrom d2 ausgeschleust werden muss.

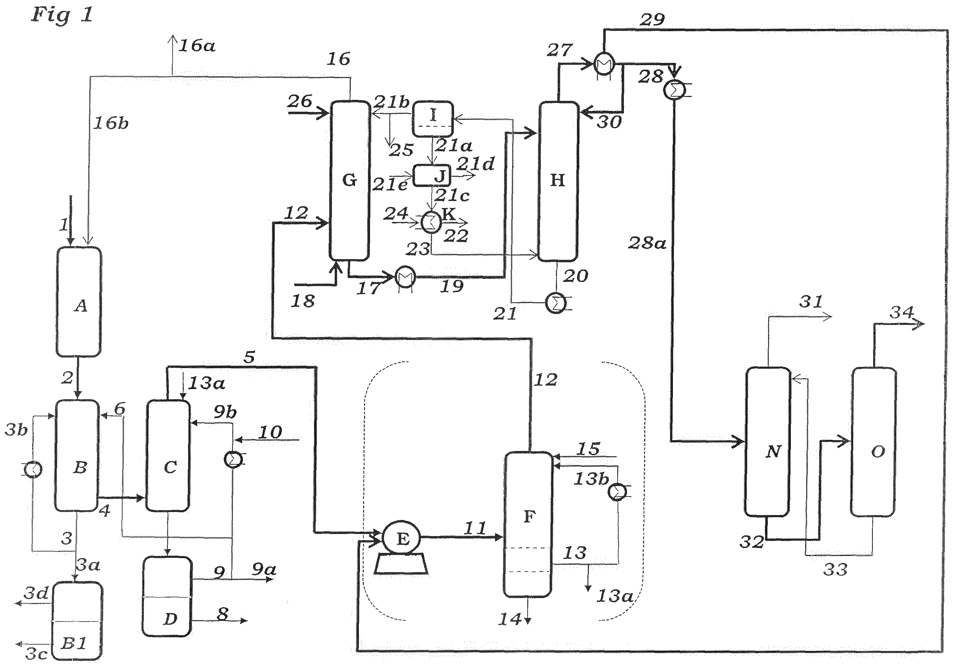

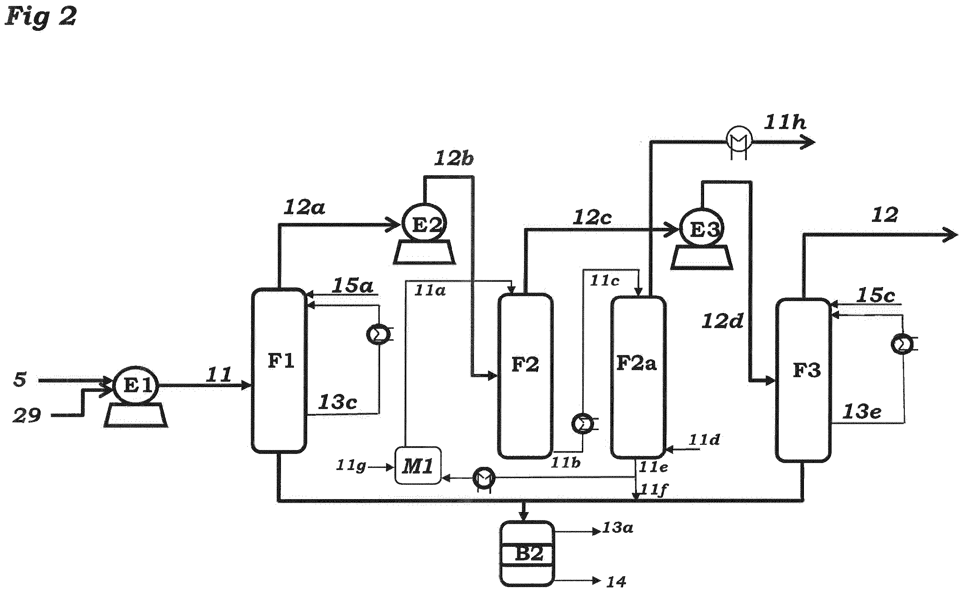

Das Gesamtverfahren mit nicht erfindungsgemäßer Durchführung des Kompressions- und Abkühlschrittes Cb) ist in Figur 1 dargestellt. Eine besonders bevorzugte Variante des Kompressions- und Abkühlschrittes gemäß der vorliegenden Erfindung ist in Figur 2 dargestellt.

Als Einsatzgasstrom werden n-Butene (1-Buten und/oder cis-/trans-2-Buten), und iso-Buten enthaltende Gasgemische eingesetzt. Ein solches Gasgemisch kann beispielsweise durch oxidative oder nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der C₄-Fraktion des Naphtha-Crackens durch Abtrennung von Butadien und iso-Buten erhalten wurde. Des Weiteren können auch Gasgemische als Eingangsgasstrom eingesetzt werden, die 1-Buten, cis-2-Buten, trans-2-Buten oder Mischungen daraus umfassen, und die durch Dimerisierung von Ethylen erhalten wurden. Ferner können als Eingangsgasstrom n-Butene enthaltende Gasgemische eingesetzt werden, die durch katalytisches Wirbelschichtcracken (Fluid Catalytic Cracking, FCC) erhalten wurden.

In einer Ausführungsform des erfindungsgemäßen Verfahrens wird das n-Butene enthaltende Ausgangsgasgemisch durch oxidative oder nicht-oxidative Dehydrierung von n-Butan erhalten. In einer bevorzugten Variante der Ausführungsform wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom und ein sauerstoffhaltiges Gas in mindestens eine Dehydrierzone (den ODH-Reaktor A) eingespeist und die in dem Gasgemisch enthaltenen Butene in Gegenwart eines Oxidehydrierungskatalysators oxidativ zu Butadien dehydriert.

In einer Ausführungsform ist bevorzugt, ein sauerstoffhaltiges Gas zu verwenden, das mehr als 10 Vol.-%, vorzugsweise mehr als 15 Vol.-% und besonders bevorzugt mehr als 20 Vol.-% molekularen Sauerstoff enthält. In einer Ausführungsform wird Luft als sauerstoffhaltiges Gas eingesetzt. Die Obergrenze für den Gehalt an molekularem Sauerstoff in dem sauerstoffhaltigen Gas beträgt dann im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, CO₂ und Wasser genannt werden. Die Menge an Inertgasen in dem sauerstoffhaltigen Gas beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff in dem sauerstoffhaltigen Gas beträgt sie im Allgemeinen 10 Vol.-% oder weniger, vorzugsweise 1 Vol.-% oder weniger.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃CO_{4,5}Ni_{2,5}Mn_{0,5}K_{0,1}Oₓ und Mo₁₂BiFe₃CO_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (la) auf:

Mo₁₂BiₐFe_{b}Co_{c}Ni_{d}CrₑX¹_{f}X²_{g}O_{y} (Ia),

mit
X¹ = Si, Mn und/oder Al,
X² = Li, Na, K, Cs und/oder Rb,
0,2 ≤ a ≤ 1,
0,5 ≤ b ≤ 10,
0 ≤ c ≤ 10,
0 ≤ d ≤ 10,
2 ≤ c + d ≤ 10,
0 ≤ e ≤ 2,
0 ≤ f ≤ 10,
0 ≤ g ≤ 0,5,
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K. Besonders bevorzugt ist ein weitgehend Cr(VI)-freier Katalysator.

Zur Durchführung der oxidativen Dehydrierung bei hohem Gesamtumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigem Gas und gegebenenfalls zusätzlichem Inertgas, Methan oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen oder Salzgemischen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490°C und bevorzugt zwischen 300 bis 450°C und besonders bevorzugt zwischen 350 und 420°C.

Aufgrund der Exothermie der ablaufenden Reaktionen kann die Temperatur in bestimmten Abschnitten des Reaktorinneren während der Reaktion höher liegen als diejenige des Wärmeaustauschmittels, und es bildet sich ein sogenannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1 bis 150°C, bevorzugt zwischen 10 bis 100°C und besonders bevorzugt zwischen 20 bis 80°C. Die Temperatur am Ende des Katalysatorbettes liegt im Allgemeinen zwischen 0 bis 100°C, vorzugsweise zwischen 0,1 bis 50°C, besonders bevorzugt zwischen 1 bis 25°C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im ODH-Reaktor A eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Eingangsgas oder Komponenten aus dem Produktgas der Reaktion reagiert. Weiterhin können die Katalysatorschichten aus Vollmaterial und/oder geträgerten Schalenkatalysatoren bestehen.

Der die oxidative Dehydrierung verlassende Produktgasstrom 2 enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Acrolein, Methylvinylketon, Styrol, Benzaldehyd, Benzoesäure, Phthalsäureanhydrid, Fluorenon, Anthrachinon und Butyraldehyd sein.

Der Produktgasstrom 2 am Reaktorausgang ist durch eine Temperatur nahe der Temperatur am Ende des Katalysatorbetts charakterisiert. Der Produktgasstrom wird dann auf eine Temperatur von 150 bis 400°C, bevorzugt 160 bis 300°C, besonders bevorzugt 170 bis 250°C gebracht. Es ist möglich, die Leitung, durch die der Produktgasstrom fließt, um die Temperatur im gewünschten Bereich zu halten, zu isolieren oder einen Wärmetauscher einzusetzen. Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes Kühlmittel kann ein Lösungsmittel, solange es in der Lage ist, die hochsiedenden Nebenprodukte aufzulösen, verwendet werden. Beispiele sind aromatische Kohlenwasserstofflösungsmittel, wie z.B. Toluol, Xylole, Diethylbenzole, Trietylbenzole, Diisopropylbenzole und Triisopropylbenzole. Besonders bevorzugt ist Mesitylen. Es können auch wässrige Lösungsmittel verwendet werden. Diese können sowohl sauer als auch alkalisch gestellt werden, wie zum Beispiel eine wässrige Lösung von Natriumhydroxid.

Anschließend wird aus dem Produktgasstrom 2 durch Abkühlung und Kompression ein Großteil der hochsiedenden Nebenkomponenten und des Wassers abgetrennt werden. Die Abkühlung erfolgt durch Inkontaktbringen mit einem Kühlmittel. Diese Stufe wird nachfolgend auch als Quench bezeichnet. Dieser Quench kann aus nur einer Stufe oder aus mehreren Stufen bestehen (beispielsweise B, C in Figur 1). Der Produktgasstrom 2 wird also direkt mit dem organischen Kühlmedium 3b und 9b in Kontakt gebracht und dadurch gekühlt. Als Kühlmedium geeignet sind wässrige Kühlmittel oder organische Lösungsmittel, bevorzugt aromatische Kohlenwasserstoffe, besonders bevorzugt Toluol, o-Xylol, m-Xylol, p-Xylol oder Mesitylen, oder Gemische daraus. Verwendet werden können auch Diethylbenzol, Trietylbenzol, Diisopropylbenzol und Triisopropylbenzol.

Bevorzugt ist ein zweistufiger Quench (umfassend die Stufen B und C gemäß Figur 1), d. h. die Stufe Ca) umfasst zwei Abkühlstufen Ca1) und Ca2), in denen der Produktgasstrom 2 mit dem organischen Lösungsmittel in Kontakt gebracht wird.

Im Allgemeinen hat das Produktgas 2, je nach Vorliegen und Temperaturniveau eines Wärmetauschers vor dem Quench B, eine Temperatur von 100 bis 440°C. Das Produktgas wird in der 1. Quenchstufe B mit dem Kühlmedium aus organischem Lösungsmittel in Kontakt gebracht. Hierbei kann das Kühlmedium durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, die das Produktgas und das Kühlmedium gemeinsam passieren. Der Kühlmitteleinlass in den Quench ist so ausgelegt, dass ein Verstopfen durch Ablagerungen im Bereich des Kühlmitteleinlasses minimiert wird.

Im Allgemeinen wird das Produktgas 2 in der ersten Quenchstufe Ca1) auf 5 bis 180°C, vorzugsweise auf 30 bis 130°C und noch mehr bevorzugt auf 60 bis 110°C gekühlt. Die Temperatur des Kühlmittelmediums 3b am Einlass kann im Allgemeinen 25 bis 200°C, bevorzugt 40 bis 120°C, insbesondere bevorzugt 50 bis 90°C betragen. Der Druck in der ersten Quenchstufe B ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Wenn größere Mengen hochsiedende Nebenprodukte im Produktgas vorhanden sind, kann es leicht zur Polymerisation von hochsiedenden Nebenprodukten und zu Ablagerungen von Feststoffen, die durch hochsiedende Nebenprodukte in diesem Verfahrensabschnitt verursacht werden, kommen. Im Allgemeinen ist die Quenchstufe B als Kühlturm ausgestaltet. Das im Kühlturm eingesetzte Kühlmedium 3b wird häufig zirkulierend eingesetzt. Der Kreislaufstrom des Kühlmediums in Liter pro Stunde, bezogen auf den Massenstrom an Butadien in Gramm pro Stunde, kann im Allgemeinen 0,0001 bis 5 l/g, bevorzugt 0,001 bis 1 l/g und besonders bevorzugt 0,002 bis 0,2 l/g betragen.

In der ersten Quenchstufe Ca1) kann eine wässrige Phase anfallen. Diese kann in einem Phasenscheider abgetrennt werden.

Die Temperatur des Kühlmediums 3 im Sumpf kann im Allgemeinen 27 bis 210°C, bevorzugt 45 bis 130°C, insbesondere bevorzugt 55 bis 95°C betragen. Da die Beladung des Kühlmediums 3 mit Nebenkomponenten im Laufe der Zeit zunimmt, kann ein Teil des beladenen Kühlmediums aus dem Umlauf als Purgestrom 3a abgezogen und die Umlaufmenge durch Zugabe von unbeladenerem Kühlmedium 6 konstant gehalten werden. Das Verhältnis von Ablaufmenge und Zugabemenge hängt von der Dampfbeladung des Produktgases und der Produktgastemperatur am Ende der erste Quenchstufe ab.

Der abgekühlte und eventuell an Nebenkomponenten abgereicherte Produktgasstrom 4 kann nun einer zweiten Quenchstufe Ca2) zugeführt werden. In dieser kann er nun erneut mit einem organischen Kühlmedium 9b in Kontakt gebracht werden.

Im Allgemeinen wird das Produktgas bis zum Gasausgang der zweiten Quenchstufe Ca2) auf 5 bis 100°C, vorzugsweise auf 15 bis 85°C und noch mehr bevorzugt auf 30 bis 70°C gekühlt. Das Kühlmittel kann im Gegenstrom zum Produktgas zugeführt werden. In diesem Fall kann die Temperatur des Kühlmittelmediums 9b am Kühlmitteleinlass 5 bis 100°C, bevorzugt 15 bis 85°C, insbesondere bevorzugt 30 bis 70°C betragen. Der Druck in der zweiten Quenchstufe C ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Die zweite Quenchstufe ist bevorzugt als Kühlturm ausgestaltet. Das im Kühlturm eingesetzte Kühlmedium 9b wird häufig zirkulierend eingesetzt. Der Kreislaufstrom des Kühlmediums 9b in Liter pro Stunde, bezogen auf den Massenstrom an Butadien in Gramm pro Stunde, kann im Allgemeinen 0,0001 bis 5 l/g, bevorzugt 0,3001 bis 1 l/g und besonders bevorzugt 0,002 bis 0,2 l/g betragen.

Aufgrund von Temperatur, Druck und Wassergehalt des Produktgases 4 kommt es in der zweiten Quenchstufe C zur Kondensation von Wasser. Dabei bildet sich eine wässrige Phase 8 aus, welche bestimmte Säuren als wasserlösliche Nebenkomponenten enthält. Diese wässrige Phase kann dann im Phasenscheider D abgezogen werden. Die Temperatur des Kühlmediums 9 im Sumpf kann im Allgemeinen 20 bis 210°C, bevorzugt 35 bis 120°C, insbesondere bevorzugt 45 bis 85°C betragen. Da die Beladung des Kühlmediums 9 mit Nebenkomponenten im Laufe der Zeit zunimmt, kann ein Teil des beladenen Kühlmediums als Purgestrom 9a aus dem Umlauf abgezogen werden, und die Umlaufmenge durch Zugabe von unbeladenem Kühlmedium 10 konstant gehalten werden.

Um einen möglichst guten Kontakt von Produktgas und Kühlmedium zu erreichen, können Einbauten in der zweiten Quenchstufe Ca2) vorhanden sein. Solche Einbauten umfassen zum Beispiel Glocken-, Zentrifugal- und/oder Siebböden, Kolonnen mit strukturierten Packungen, z. B. Blechpackungen mit einer spezifischen Oberfläche von 100 bis 1000 m²/m³ wie Mellapak® 250 Y, und Füllkörperkolonnen.

Die Lösungsmittel-Umläufe der beiden Quenchstufen können sowohl voneinander getrennt als auch miteinander verbunden sein. So kann beispielsweise der Strom 9a dem Strom 3b zugeführt werden oder diesen ersetzen. Die gewünschte Temperatur der Umlaufströme kann über geeignete Wärmetauscher eingestellt werden.

In einer bevorzugten Ausführungsform der Erfindung wird also die Abkühlstufe Ca) zweistufig durchgeführt, wobei das mit Nebenkomponenten beladene Lösungsmittel der zweiten Stufe Ca2) in die erste Stufe Ca1) geführt wird. Das der zweiten Stufe Ca2) entnommene Lösungsmittel enthält weniger Nebenkomponenten als das der ersten Stufe Ca1) entnommene Lösungsmittel.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werden durch weitere bauliche Maßnahmen, wie beispielsweise weitere Gaswäschen, aus dem Produktgas entfernt werden.

Es wird ein Gasstrom 5 erhalten, der n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Lösungsmittels enthält. Weiterhin können in diesem Gasstrom 5 Spuren von hochsiedenden Komponenten verbleiben, welche im Quench nicht quantitativ abgetrennt wurden.

Anschließend wird der Gasstrom b aus dem Abkühlschritt Ca), der an hochsiedenden Nebenkomponenten abgereichert ist, im Schritt Cb) in mindestens einer Kompressionsstufe Cba) und bevorzugt in mindestens einer Abkühlstufe Cbb) durch Inkontaktbringen mit einem organischen Lösungsmittel als Abkühlmittel abgekühlt.

In Verfahren nach dem Stand der Technik wird, wie in Figur 1 dargestellt, der Produktgasstrom 5 aus dem Lösungsmittel-Quench in mindestens einer Kompressionsstufe E komprimiert und nachfolgend in dem Kühlapparat F mit dem umlaufenden Kühlmedium 13 weiter abgekühlt, wobei mindestens ein Kondensatstrom 14 entsteht. Es verbleibt ein Gasstrom 12 enthaltend Butadien, 1-Buten, 2-Butene, gegebenenfalls Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase. Weiterhin kann dieser Produktgasstrom noch Spuren von hochsiedenden Komponenten enthalten.

Die Kompression und Kühlung des Gasstroms 5 kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60°C abgekühlt wird. Der Kondensatstrom kann somit bei mehrstufiger Kompression auch mehrere Ströme umfassen. Der Kondensatstrom besteht zu großen Teilen aus Wasser und dem im Quench verwendeten Lösungsmittel. Beide Ströme (wässrige und organische Phase) können daneben in geringem Umfang Nebenkomponenten wie Leichtsieder, C₄-Kohlenwasserstoffe, Oxygenate und Kohlenstoffoxide enthalten.

Um den durch Kompression von Strom 5 entstandenen Strom 11 zu kühlen und/oder um weitere Nebenkomponenten aus dem Strom 11 zu entfernen, wird der komprimierte Strom 11 im Apparat F mit dem umlaufenden Abkühlmittel 13 in Kontakt gebracht. Da die Beladung dieses Abkühlmittels 13 mit Nebenkomponenten im Laufe der Zeit zunimmt, kann ein Teil des beladenen Abkühlmittels aus dem Umlauf abgezogen werden und die Umlaufmenge des Abkühlmittels durch Zugabe von unbeladenem Lösungsmittel 15 konstant gehalten werden.

Das Lösungsmittel 15, welches als Abkühlmittel zugegeben wird, kann ein wässriges Kühlmittel oder ein organisches Lösungsmittel sein. Bevorzugt sind aromatische Kohlenwasserstoffe, besonders bevorzugt sind Toluol, o-Xylol, m-Xylol, p-Xylol, Diethylbenzol, Trietylbenzol, Diisopropylbenzol, Triisopropylbenzol, Mesitylen oder Gemische daraus. Besonders bevorzugt ist Mesitylen.

Ein Teil 13a des Abkühlmittels 13 kann in den Kreislaufstrom 3b und/oder 9b des Lösungsmittel-Quenches zurückgeführt werden. Dadurch können die im Abkühlmittel 13a absorbierten C₄-Komponenten wieder in den Gasstrom gebracht und damit die Ausbeute erhöht werden.

In der in Figur 2 dargestellten Variante des erfindungsgemäßen Verfahrens sind die in Figur 1 dargestellten Apparate E und F durch die Kompressoren E1, E2 und E3, die Kühlkolonnen F1 und F3 sowie die Waschkolonne F2 und die Regenerationskolonne F2a ersetzt. Der Produktgasstrom 5 aus dem Lösungsmittel-Quench wird in einer ersten Kompressionsstufe E1 komprimiert und nachfolgend in der ersten Kühlkolonne F1 mit dem umlaufenen Abkühlmittel 13c abgekühlt, wobei am Kolonnensumpf ein zweiphasiges Gemisch aus Lösungsmittel und wässrigem Kondensat erhalten wird, das dem Phasenscheider B2 zugeführt wird. Der am Kolonnenkopf der Kühlkolonne F1 erhaltene komprimierte und gekühlte Produktgasstrom 12a wird in einer zweiten Kompressionsstufe E2 weiter komprimiert und als stärker komprimierter Produktgasstrom 12b der Waschkolonne F2 zugeführt und mit einem Gemisch aus Wasser und Essigsäure als Waschlösungsmittelstrom 11a von Aldehyden frei gewaschen und dabei ebenfalls abgekühlt, wobei am Kolonnenkopf ein an Aldehyden stark abgereicherter Produktgasstrom 12c und am Kolonnensumpf ein mit Aldehyden beladener Waschlösungsmittelstrom 11 b erhalten werden. Der mit Aldehyden beladene Waschlösungsmittelstrom 11 b wird erhitzt und als Strom 11c der Regenerationskolonne F2a zugeführt. In der Regenerationskolonne F2a wird der erhitzte Waschlösungsmittelstrom 11 c mit Stickstoff 11 d gestrippt, wobei am Kolonnensumpf ein regenerierter Waschlösungsmittelstrom 11e und am Kolonnenkopf ein die Aldehyde und in geringen Mengen C4-Kohlenwasserstoffe enthaltender Stickstoffstrom 11 h erhalten werden. Der beladene Stickstoffstrom 11 h kann einer Verbrennung zugeführt werden. Von dem regenerierten Waschlösungsmittelstrom 11e wird ein Purgestrom 11f abgezogen und zusammen mit dem Sumpfstrom aus F1 und F3 dem Phasenscheider B2 (der Abwasserbehandlung) zugeführt, wobei dieser Purgestrom in dem Mischer M1 durch frisches Waschlösungsmittel 11g ersetzt wird.

Der an Aldehyden abgereicherte Produktgasstrom 12c wird in einer dritten Kompressionsstufe E3 weiter komprimiert und als stärker komprimierter Produktgasstrom 12d nachfolgend in der weiteren Kühlkolonne F3 mit dem umlaufenden Abkühlmittel 13e abgekühlt, wobei am Kolonnenkopf ein stark komprimierter, abgekühlter und im Wesentlichen Aldehyd-freier Produktstrom 12 und am Kolonnensumpf ein zweiphasiges Gemisch aus Abkühlmittel und wässrigem Kondensat erhalten wird, das dem Phasenscheider B2 zugeführt wird. Das in dem Phasenscheider B2 abgetrennte Abkühlmittel 13f kann in den Kreislaufstrom 3b und/oder 9b des Lösungsmittel-Quenches zurückgeführt werden. Dadurch können die im Abkühlmittel 13a absorbierten C₄-Komponenten wieder in den Gasstrom gebracht und damit die Ausbeute erhöht werden. Das abgezogene Abkühlmittel 13a wird durch frisches Abkühlmittel 15 ersetzt. Die wässrige Kondensatphase 14 kann einer Abwasserbehandlung zugeführt werden.

Geeignete Verdichter E1, E2 und E3 sind beispielsweise Turbo-, Drehkolben- und Hubkolbenverdichter. Die Verdichter können beispielsweise mit einem Elektromotor, einem Expander oder einer Gas oder Dampfturbine angetrieben werden. Der Eingangsdruck in die erste Kompressorstufe beträgt 0,5 bis 3 bar absolut, bevorzugt 1 bis 2 bar absolut. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt in mit Kühlmittel gespülten Wärmetauschern oder organischen Quenchstufen, die beispielsweise als Rohrbündel-, Spiral oder Plattenwärmetauscher ausgeführt sein können. Geeignete Kühlmittel können wässrige oder die oben genannten organischen Lösungsmittel sein. Als Kühlmittel in den Wärmetauschern kommen dabei Kühlwasser oder Wärmeträgeröle oder organische Lösungsmittel zum Einsatz. Daneben wird bevorzugt Luftkühlung unter Einsatz von Gebläsen eingesetzt.

Der Butadien, n-Butene, gegebenenfalls Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase und gegebenenfalls Spuren von Nebenkomponenten enthaltende Gasstrom 12 wird als Ausgangsstrom der weiteren Aufbereitung zugeführt.

In einem Schritt D) werden nicht kondensierbare und leicht siedenden Gasbestandteile, umfassend Sauerstoff, leicht siedenden Kohlenwasserstoffe (Methan, Ethan, Ethen, Propan, Propen), Kohlenstoffoxide und Inertgase in einer Absorptionskolonne G als Gasstrom 16 aus dem Prozessgasstrom 12 durch Absorption der C₄-Kohlenwasserstoffe in einem hochsiedenden Absorptionsmittel (21 b und/oder 26) und nachfolgender Desorption der C₄-Kohlenwasserstoffe abgetrennt. Vorzugsweise umfasst der Schritt D), wie in Figur 1 dargestellt, die Schritte Da1), Da2) und Db):
Da1) Absorption der C₄-Kohlenwasserstoffe umfassend Butadien und n-Butene in einem hochsiedenden Absorptionsmittel (21 b und/oder 26), wobei ein mit C₄-Kohlenwasserstoffen beladener Absorptionsmittelstrom und der Gasstrom 16 erhalten werden,
Da2) Entfernung von Sauerstoff aus dem mit C₄-Kohlenwasserstoffen beladenen Absorptionsmittelstrom aus Schritt Da1) durch Strippung mit einem nicht kondensierbaren Gasstrom 18, wobei ein mit C₄-Kohlenwasserstoffen beladener Absorptionsmittelstrom 17 erhalten wird, und
Db) Desorption der C₄-Kohlenwasserstoffe aus dem beladenen Absorptionsmittelstrom, wobei ein C₄-Produktgasstrom 27 erhalten wird, der im Wesentlichen aus C₄-Kohlenwasserstoffen besteht.

Dazu wird in der Absorptionsstufe G der Gasstrom 12 mit einem Absorptionsmittel in Kontakt gebracht und werden die C₄-Kohlenwasserstoffe in dem Absorptionsmittel absorbiert, wobei ein mit C₄-Kohlenwasserstoffen beladenes Absorptionsmittel und ein die übrigen Gasbestandteile enthaltendes Abgas 16 erhalten werden. In einer Desorptionsstufe H werden die C₄-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom12 zugeführt. Im oberen Bereich der Absorptionskolonne wird das hochsiedende Absorptionsmittel (21 b und/oder 26) aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁- bis C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%.

In einer bevorzugten Ausführungsform wird in der Absorptionsstufe Da1) das gleiche Lösungsmittel wie in der Abkühlstufe Ca) eingesetzt.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. Bevorzugt sind aromatische Kohlenwasserstoffe, besonders bevorzugt Toluol, o-Xylol, p-Xylol und Mesitylen, oder Gemische daraus. Verwendet werden können auch Diethylbenzol, Triethylbenzol, Diisopropylbenzol und Triisopropylbenzol.

Am Kopf der Absorptionskolonne G wird ein Strom 16 abgezogen, der im Wesentlichen das Strippgas, Sauerstoff, leicht siedende Kohlenwasserstoffe (Methan, Ethan, Ethen, Propan, Propen), gegebenenfalls C₄-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten C₄-Kohlenwasserstoffgehalt und Sauerstoffgehalt einstellen.

Am Sumpf der Absorptionskolonne werden durch die Spülung mit einem Gas 18 Reste von im Absorptionsmittel gelöstem Sauerstoff ausgetragen. Der verbleibende Sauerstoffanteil soll so klein sein, dass der, die Desorptionskolonne verlassende und Butan, Buten sowie Butadien enthaltende, Strom 27 nur noch maximal 100 ppm Sauerstoff enthält.

Das Ausstrippen des Sauerstoffs in Schritt Db) kann in jeder beliebigen, dem Fachmann bekannten geeigneten Kolonne durchgeführt werden. Das Strippen kann durch einfaches Durchleiten von nicht kondensierbaren Gasen, vorzugsweise nicht oder nur schwach im Absorptionsmittelstrom 21 b und/oder 26 absorbierbaren Gase wie Stickstoff oder Methan, durch die beladene Absorptionslösung erfolgen. Mit ausgestrippte C₄-Kohlenwasserstoffe werden im oberen Teil der Kolonne G zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil gleich ist, kann diese direkte Kopplung erfolgen. Geeignete Stippkolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der Strom 17 kann gegebenenfalls abgekühlt oder erhitzt werden und tritt als Strom 19 in die Desorberkolonne ein. Die Eintrittsstelle liegt im Allgemeinen 0 bis 10 theoretische Trennstufen, bevorzugt 2 bis 8 theoretische Trennstufen, besonders bevorzugt 3 bis 5 theoretische Trennstufen unterhalb des Kolonnenkopfes.

Das in der Desorptionsstufe regenerierte Absorptionsmittel wird als Strom 20 zusammen mit dem kondensierten Wasser aus der Desorptionskolonne H entnommen. Dieses zweiphasige Gemisch kann in einem Wärmetauscher abgekühlt werden und als Strom 21 in einem Dekanter I in einen wässrigen Strom 21 a und einen Absorptionsmittelstrom 21 b aufgetrennt werden. Der Absorptionsmittelstrom 21 b wird wieder der Absorptionskolonne G zugeführt, während der wässrige Strom 21 a in einer Kolonne J durch Erhitzen und gegebenenfalls durch Strippen mit einem nicht kondensierbaren Gas 21 e, vorzugsweise Stickstoff, von Nebenkomponenten wie zum Beispiel Acetaldehyd, Acrolein und Metacrolein befreit werden kann. Der gereinigte Wasserstrom 21 c wird in einem Verdampfer K verdampft wird und als Strippdampf-Strom 23 wieder in die Desorptionskolonne H eingespeist. In den Verdampfer K kann zusätzlich noch Frischwasser als Strom 24 eingespeist werden. Ein Teil des Stroms 21 c kann als Strom 22 entnommen und der Abwasserbehandlung zugeführt werden.

Im Prozessgasstrom befindliche Leichtsieder wie beispielsweise Ethan oder Propan sowie schwersiedende Komponenten wie Benzaldehyd, Maleinsäureanhydrid und Phthalsäureanhydrid können sich im Absorptionsmittelkreislaufstrom anreichern. Um die Anreicherung zu begrenzen, kann ein Purgestrom 25 abgezogen werden.

Der im Wesentlichen aus n-Butan, n-Butenen und Butadien bestehende C₄-Produktgasstrom 27 enthält im Allgemeinen 20 bis 80 Vol.-% Butadien, 0 bis 80 Vol.-% n-Butan, 0 bis 10 Vol.-% 1-Buten und 0 bis 50 Vol.-% 2-Butene, wobei die Gesamtmenge 100 Vol.-% ergibt. Weiterhin können geringe Mengen an iso-Butan enthalten sein.

Ein Teil des kondensierten, hauptsächlich C₄-Kohlenwasserstoffe enthaltenen Kopfaustrags der Desorptionskolonne wird als Strom 30 in den Kolonnenkopf zurückgeführt, um die Trennleistung der Kolonne zu erhöhen.

Die Desorptionsstufe kann in jeder beliebigen, dem Fachmann bekannten geeigneten Desorptionskolonne durchgeführt werden. Die Desorption kann durch Absenken des Druckes und/oder Erhitzen der Desorptionsstufe erfolgen. Die Desorptionsstufe kann durch Zuführen eines heißen Mediums - wie zum Beispiel Wasserdampf - oder durch Eigendampf, der z.B. durch Teilverdampfen des Absorbens im Sumpf der Desorberkolonne erzeugt wird, erhitzt werden.

Geeignete Desorptionskolonnen sind z.B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 m²/m³ wie Mellapak® 250 Y, und Füllkörperkolonnen. Bevorzugt weist die Desorptionskolonne 5 bis 30, besonders bevorzugt 10 bis 20 theoretische Böden auf.

Der gasförmige, C₄-Kohlenwasserstoffe enthaltende Strom 29, der im Kopfkondensator der Desorptionskolonne H nicht kondensiert, wird zum Kompressor E1 zurückgeführt.

In einer Ausführung wird der Strom 28, bevor er in den Verdampfer eintritt, in einer Flüssig-Flüssig-Wäsche mit Polyalkoholen wie Ethylenglykol und Glycerin oder auch Methanol gewaschen und so Furan teilweise abgetrennt. In einer weiteren Ausführung kann der Strom 28 zuvor in einer Flüssig-Flüssig-Wäsche mit Wasser von anderen Nebenkomponenten wie Aldehyden befreit werden.

Der weitgehend von Nebenkomponenten befreite Strom 28a wird durch Extraktivdestillation im Schritt E) mit einem für Butadien selektiven Lösungsmittel in einen Butadien und das selektive Lösungsmittel enthaltenden Stoffstrom 33 und einen Butane und n-Butene enthaltenden Stoffstrom 32 aufgetrennt.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250°C, insbesondere bei einer Temperatur im Bereich von 110 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom 31 der Extraktivdestillationskolonne N enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol.-% n-Butan, 0 bis 50 Vol.-% 2-Buten und 0 bis 3 Vol.-% weitere Bestandteile wie Isobutan, Isobuten, Propan, Propen und C₅⁺-Kohlenwasserstoffe.

Der im Wesentlichen aus n-Butan und 2-Buten und gegebenenfalls Methan bestehende Strom 31 kann ganz oder teilweise oder auch nicht in den C₄-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden. Weiterhin kann der Strom auch einer weiteren Aufarbeitung zugeführt werden, um Butane und Butene voneinander zu trennen und die Butene ganz oder teilweise in die Oxidehydrierung zurückzuführen. Der Strom kann auch in die Maleinsäureanhydrid-Produktion gehen. Der abgetrennte Butan-Strom kann ganz oder teilweise einer Butan-Dehydrierung zugeführt werden.

In einem Schritt F) wird der Butadien und das selektive Lösungsmittel enthaltende Stoffstrom 33 in einen im Wesentlichen aus dem selektiven Lösungsmittel bestehenden Stoffstrom 34 und einen Butadien enthaltenden Stoffstrom 35 destillativ aufgetrennt.

Der am Sumpf der Extraktivdestillationskolonne N gewonnene Stoffstrom 32 enthält im Allgemeinen das Extraktionsmittel, Wasser, Butadien und in geringen Anteilen Butene und Butan und wird einer Destillationskolonne O zugeführt. In dieser kann über Kopf oder als Seitenabzug Butadien als Strom 34 gewonnen werden. Am Sumpf der Destillationskolonne fällt ein Extraktionsmittel und gegebenenfalls Wasser enthaltender Stoffstrom 33 an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom 33 wird in die Extraktivdestillation zurückgeleitet.

Falls das Butadien über einen Seitenabzug gewonnen wird, wird die so abgezogene Extraktionslösung in eine Desorptionszone überführt, wobei aus der Extraktionslösung das Butadien nochmals desorbiert und rückgewaschen wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300°C, insbesondere im Bereich von 150 bis 200°C und einer Kopftemperatur im Bereich von 0 bis 70°C, insbesondere im Bereich von 10 bis 50°C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der Wertproduktstrom 34 enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Gegenstand der Erfindung ist auch eine Vorrichtung zur Durchführung des vorstehend beschriebenen Verfahrens umfassend
I) Mittel zur Bereitstellung eines n-Butene enthaltenden Einsatzgasstroms a und eines sauerstoffhaltigen Gases;
II) Reaktor umfassend eine oxidative Dehydrierzone zur oxidativen Dehydrierung von n-Butenen zu Butadien;
III) Mittel zur Abkühlung des Produktgasstroms der oxidativen Dehydrierung in einer oder mehreren Abkühlstufen durch Inkontaktbringen mit einem im Kreis geführten Kühlmittel;
IV) Mittel zur Kompression des Produktgasstroms der oxidativen Dehydrierung in mindestens einer Kompressionsstufe und gegebenenfalls mindestens einer Abkühlstufe, umfassend mindestens eine Waschstufe zum Waschen des komprimierten Produktgasstroms;
V) Mittel zur Abtrennung von nicht kondensierbaren und leicht siedenden Gasbestandteilen aus dem Produktgasstrom der oxidativen Dehydrierung durch Absorption von C4-Kohlenwasserstoffen umfassend Butadien und n-Butene in einem Absorptionsmittel;
VI) Mittel zur Desorption von C4-Kohlenwasserstoffen aus dem beladenen Absorptionsmittelstrom umfassend eine Desorptionskolonne;
VII) Mittel zum Waschen eines C₄-Produktgasstroms d1 mit einem wässrigen Strom;
VIII) optional Mittel zur Auftrennung des C₄-Produktstroms e1 durch Extraktivdestillation;
IX) optional Mittel zur Destillation eines Butadien und ein für Butadien selektives Lösungsmittel enthaltenden Stoffstroms.

### Beispiel

Der in den Figuren 1 und 2 dargestellte Prozess wurde numerisch simuliert. Das verwendete Simulationsprogramm entspricht dem kommerziell erhältlichen Programm Aspen Plus^{®} zur Prozesssimulation.

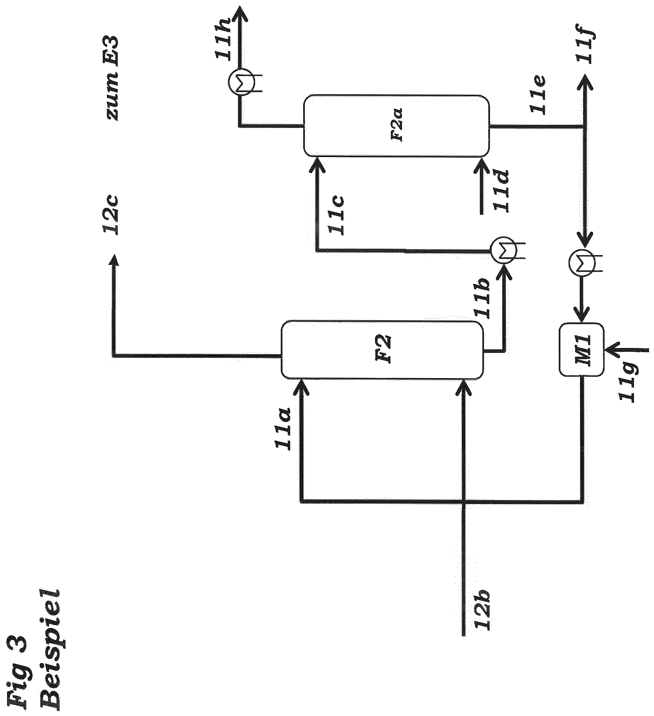

Die Zusammensetzung der in Figur 3 dargestellten Ströme ist in Tabelle 1 wiedergegeben.

Für die Waschkolonne F2 nimmt die Simulation einen Druck von 5,2 bar absolut und 10 theoretischen Stufen an. Das die zweite Kompressionsstufe E2 verlassende Prozessgasgemisch 12b tritt mit 85 °C und einem Aldehydgehalt von insgesamt 8400 ppm in die Waschkolonne ein. Der Kopfdruck der Kolonne beträgt 5 bar absolut. In der Waschkolonne strömt der Prozessgasstrom im Gegenstrom zum von oben zugeführten Waschstrom 11a aus einem 1 : 1-Gemisch von Wasser und Essigsäure. Das Massenverhältnis von Waschstrom 11a zu Prozessgasstroms 12b beträgt 2 : 1.

Der beladene Waschlösungsstrom 11 b wird in einem Wärmetauscher erhitzt und tritt mit einer Temperatur von 85 °C in die Regenerationskolonne F2a, wo er mit einem Stickstoffstrom 11 d gestrippt wird, der in die F2a mit einer Temperatur von 35 °C eintritt. Für die Regenerationskolonne F2a nimmt die Simulation Atmosphärendruck (1,013 bar) und 5 theoretischen Stufen an. Das Massenverhältnis Waschlösungsstrom 11 c zu Stickstoffstrom 11 d beträgt 30 : 1. Der regenerierte Waschlösungsstrom 11 e tritt mit einer Temperatur von 67 °C aus der Regenerationskolonne aus. Der Strom 11e wird, nach Abtrennung eines Purgestroms 11f, auf 20 °C abgekühlt, auf 5,3 bar komprimiert und in die Waschkolonne F2 zurückgeführt.

Der mit einer Temperatur von 22 °C die Waschkolonne F2 über Kopf verlassende Produktstrom 21c weist einen Aldehydgehalt von nur noch 200 ppm auf. Der Butadien-Verlust beträgt bei diesen Betriebsbedingungen ca. 2%.

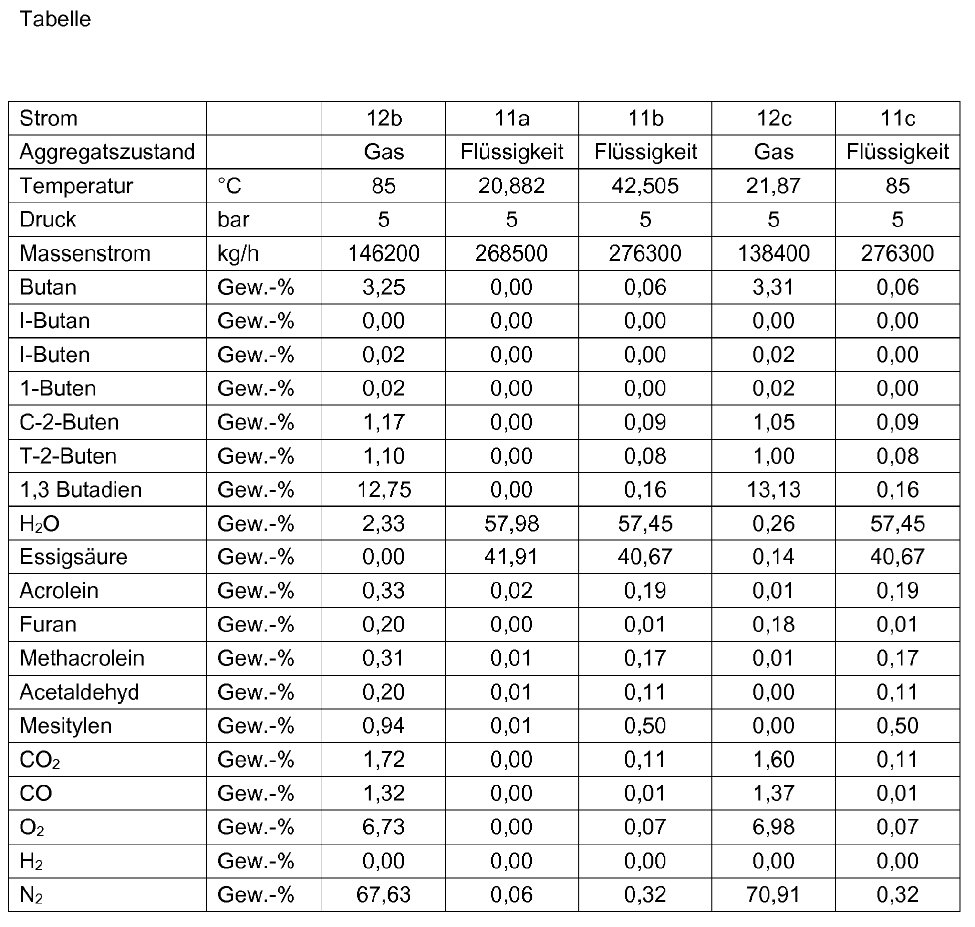

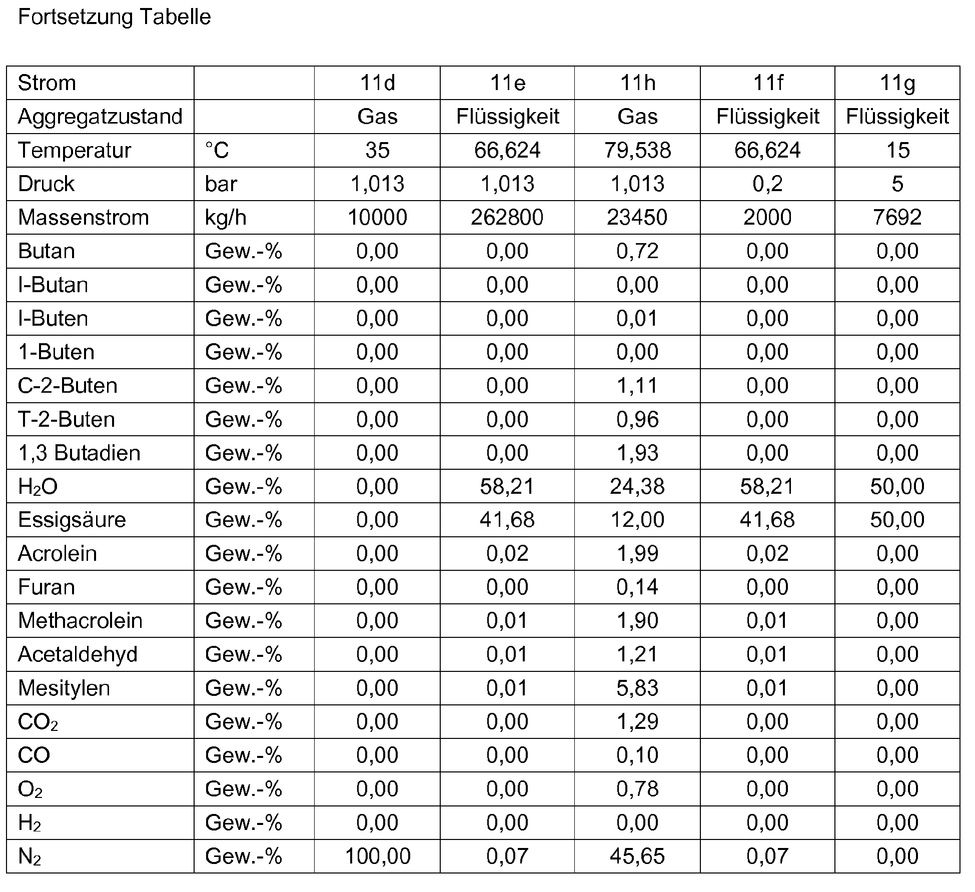

## Patentansprüche

1. Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, gegebenenfalls Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b in einer oder mehreren Abkühlstufen durch Inkontaktbringen mit einem im Kreis geführten Kühlmittel, wobei Wasserdampf und zumindest ein Teil der hochsiedenden Nebenkomponenten kondensieren;
Cb) Kompression des verbleibenden Produktgasstroms b in mindestens einer Kompressionsstufe Cba) und Abkühlung des komprimierten Gasstroms b in mindestens einer Abkühlstufe Cbb) durch Inkontaktbringen mit einem Abkühlmittel, wobei ein wässriges Kondensat c1 b und ein Gasstrom c2 enthaltend Butadien, n-Butene, Wasserdampf, gegebenenfalls Sauerstoff, leicht siedende Kohlenwasserstoffe, gegebenenfalls Kohlenstofffoxide und gegebenenfalls Inertgase erhalten werden;
Da) Abtrennung von nicht kondensierbaren und leicht siedenden Gasbestandteilen umfassend gegebenenfalls Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein Nebenkomponenten enthaltender C₄-Produktgasstrom d1 erhalten wird;
**dadurch gekennzeichnet, dass** Schritt Cb) eine Waschstufe umfasst, wobei in der Waschstufe aus dem komprimierten Produktgasstrom b durch Waschen mit einer Waschlösung aus Wasser und Essigsäure Aldehyde abgetrennt werden.

2. Verfahren nach Anspruch 1, **dadurch gekennzeichnet, dass** die in Schritt Cb) eingesetzte Waschlösung 30 bis 95 Gew.-% Wasser und 5 bis 70 Gew.-% Essigsäure enthält.

3. Verfahren nach Anspruche 1 oder 2, **dadurch gekennzeichnet, dass** die Stufe Cb) mehrere Kompressionsstufen Cba1) bis Cban) und mehrere Abkühlstufen Cbb1) bis Cbbn) umfasst, wobei auf jede Kompressionsstufe eine Abkühlstufe folgt, und mindestens eine der Abkühlstufen Cbb1) bis Cbbn) als Waschstufe ausgestaltet ist.

4. Verfahren nach Anspruch 3, **dadurch gekennzeichnet, dass** die Stufe Cb) drei Kompressionsstufen Cba1) bis Cba3) und drei Abkühlstufen Cbb1) bis Cbb3) umfasst, wobei die mittlere Abkühlstufen Cbb2) als Waschstufe ausgestaltet ist.

5. Verfahren nach Anspruch 1 oder 2, **dadurch gekennzeichnet, dass** die Stufe Cb) eine separate Waschstufe und eine separate Abkühlstufe umfasst, wobei die separate Waschstufe direkt vor oder direkt nach der separaten Abkühlstufe durchgeführt wird.

6. Verfahren nach einem der Ansprüche 1 bis 5, **dadurch gekennzeichnet, dass** in den Abkühlstufen Cbb), die nicht als Waschstufe ausgebildet sind, ein aromatischer Kohlenwasserstoff als Abkühlmittel eingesetzt wird.

7. Verfahren nach Anspruch 6, **dadurch gekennzeichnet, dass** der aromatische Kohlenwasserstoff Mesitylen ist.

8. Verfahren nach einem der Ansprüche 1 bis 7, **dadurch gekennzeichnet, dass** die die Waschstufe verlassende, mit Aldehyden beladene Waschlösung durch Strippen mit einem Gas, bevorzugt mit einem Inertgas regeneriert wird.

9. Verfahren nach Anspruch 8, **dadurch gekennzeichnet, dass** das Inertgas Stickstoff ist.

10. Verfahren nach einem der Ansprüche 1 bis 9, **dadurch gekennzeichnet, dass** die Aldehyde ausgewählt sind aus der Gruppe bestehend aus Acetaldehyd, Acrolein und Methacrolein.

11. Verfahren nach einem der Ansprüche 1 bis 10, **dadurch gekennzeichnet, dass** die Stufe Ca) zweistufig in zwei Stufen Ca1) und Ca2) durchgeführt wird.

12. Verfahren nach einem der Ansprüche 1 bis 11, **dadurch gekennzeichnet, dass** der in Schritt Da) abgetrennte Gasstrom d2 zumindest teilweise in Schritt B) zurückgeführt wird.

13. Verfahren nach einem der Ansprüche 1 bis 12, **dadurch gekennzeichnet**, das Schritt Da) die Schritte Da1), Da2) und Db) umfasst:
Da1) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Da2) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Db) Desorption der C₄-Kohlenwasserstoffe aus dem beladenen Absorptionsmittelstrom, wobei ein C₄-Produktgasstrom d1 erhalten wird, der weniger als 100 ppm Sauerstoff umfasst.

14. Verfahren nach einem der Ansprüche 1 bis 13, **dadurch gekennzeichnet, dass** das in Schritt Da) eingesetzte Absorptionsmittel ein aromatisches Kohlenwasserstofflösungsmittel ist.

15. Verfahren nach Anspruch 2, **dadurch gekennzeichnet, dass** die in Schritt Cb) eingesetzte Waschlösung 30 bis 70 Gew.-% Wasser und 30 bis 70 Gew.-% Essigsäure enthält.

16. Verfahren nach Anspruch 15, **dadurch gekennzeichnet, dass** die in Schritt Cb) eingesetzte Waschlösung 40 bis 60 Gew.-% Wasser und 40-60 Gew.-% Essigsäure enthält.

17. Verfahren nach einem der Ansprüche 1 bis 16, **dadurch gekennzeichnet, dass** das Waschen und Abkühlen nach einer oder mehreren Kompressionsstufen in 2 Schritten hintereinander als separate Abkühlstufe gefolgt von einer separaten Waschstufe ausgeführt wird.

18. Verfahren nach einem der Ansprüche 1 bis 16, **dadurch gekennzeichnet, dass** das Waschen und Abkühlen nach einer oder mehreren Kompressionsstufen in 2 Schritten hintereinander als separate Waschstufe gefolgt von einer separaten Abkühlstufe ausgeführt wird.

19. Vorrichtung zur Durchführung des Verfahrens nach einem der Ansprüche 1 bis 18 umfassend
I) Mittel zur Bereitstellung eines n-Butene enthaltenden Einsatzgasstroms a und eines sauerstoffhaltigen Gases;
II) Reaktor umfassend eine oxidative Dehydrierzone zur oxidativen Dehydrierung von n-Butenen zu Butadien;
III) Mittel zur Abkühlung des Produktgasstroms der oxidativen Dehydrierung in einer oder mehreren Abkühlstufen durch Inkontaktbringen mit einem im Kreis geführten Kühlmittel;
IV) Mittel zur Kompression des Produktgasstroms der oxidativen Dehydrierung in mindestens einer Kompressionsstufe und gegebenenfalls mindestens einer Abkühlstufe, umfassend mindestens eine Waschstufe zum Waschen des komprimierten Produktstroms;
V) Mittel zur Abtrennung von nicht kondensierbaren und leicht siedenden Gasbestandteilen aus dem Produktgasstrom der oxidativen Dehydrierung durch Absorption von C₄-Kohlenwasserstoffen umfassend Butadien und n-Butene in einem Absorptionsmittel;
VI) Mittel zur Desorption von C₄-Kohlenwasserstoffen aus dem beladenen Absorptionsmittelstrom umfassend eine Desorptionskolonne;
VII) Mittel zum Waschen eines C₄-Produktgasstroms d1 mit einem wässrigen Strom;
VIII Mittel zur Auftrennung des C₄-Produktstroms e1 durch Extraktivdestillation;
IX) Mittel zur Destillation eines Butadien und ein für Butadien selektives Lösungsmittel enthaltenden Stoffstroms.
